# EUROPÄISCHE PATENTSCHRIFT

(11) **EP 1 191 946 B1**
(45) Veröffentlichungstag und Bekanntmachung des Hinweises auf die Patenterteilung: **11.05.2011**
(21) Anmeldenummer: 00938785.3
(22) Anmeldetag: 13.06.2000
(51) Int. Cl.: A61K 49/00

(54) **DENTALES ABFORM- ODER FILMMATERIAL UND ABBILDUNGSVERFAHREN FÜR INTRAORALE DIAGNOSEZWECKE**
DENTAL MOULDING- OR FILMMATERIALS AND IMAGING METHOD FOR INTRAORAL DIAGNOSTIC PURPOSES
MATERIAUX DENTALES DE MODELAGE OU DE FILM ET PROCEDES D'IMAGERIE AUX FINS DE DIAGNOSTIQUE INTRA-ORAL

(30) Priorität: 11.06.1999 DE 19926728
(43) Veröffentlichungstag der Anmeldung: 03.04.2002
(73) Patentinhaber: 3M ESPE AG, 82229 Seefeld (DE)
(72) Erfinder: GASSER, Oswald, D-82229 Seefeld (DE); GUGGENBERGER, Rainer, D-82211 Herrsching (DE); GANGNUS, Bernd, D-82346 Andechs (DE); HÄBERLEIN, Ingo, D-82362 Weilheim (DE)
(74) Vertreter: Brem, Roland
(86) Internationale Anmeldenummer: PCT/EP2000/005418
(87) Internationale Veröffentlichungsnummer: WO 2001/012237

(56) Entgegenhaltungen:
- EP-A- 0 304 871
- WO-A-95/07286
- DE-A- 1 745 810
- DE-A- 19 753 456
- DATABASE CHEMABS [Online] CHEMICAL ABSTRACTS SERVICE, COLUMBUS, OHIO, US; YAMAMOTO, KENJI ET AL: "Preparation of peptide-substituted coumarins as fluorescent substrates fo determination of Lys-gingipain activity" retrieved from STN Database accession no. 131:144857 XP002156957 & JP 11 228597 A (TAIHO PHARAMCEUTICAL CO., LTD., JAPAN) 24. August 1999 (1999-08-24)

## Beschreibung

Die Erfindung betrifft verformbare, härtbare oder filmbildende dentale Abform - oder Filmmaterialien für die intraorale Diagnostik, welche diagnostisch nutzbare Zusatzstoffe enthalten, wie es in den Ansprüchen beschrieben ist. Ferner betrifft die Erfindung Verfahren zur Herstellung von Abbildungen für intraorale orts- und stoffspezifische Diagnosezwecke sowie Verfahren für die multiple sowie orts- und stoffspezifische Befunderhebung unter Verwendung der diagnostisch nutzbare Zusatzstoffe enthaltenden härtbaren oder filmbildenden dentalen Abform- oder Filmmaterialen. Derartige Zusatzstoffe ermöglichen dem Fachmann die Herstellung von Abbildungen für den intraoral orts- und stoffspezifischen Nachweis von pathogenen Substanzen und/oder von Mikroorganismen oder zum intraoral orts- und stoffspezifischen Nachweis von Substanzen, die auf Munderkrankungen oder Heilungsprozesse hinweisen.

Insbesondere betrifft die Erfindung dentale Abformmaterialien für die intraorale Diagnostik, welche diagnostisch nutzbare Zusatzstoffe enthalten, sowie ein Verfahren zum Aufbringen diagnostisch nutzbarer Zusatzstoffe auf ausgehärtete Abformmassen, wobei die diagnostisch wirksamen Zusatzstoffe zum intraoral orts- und stoffspezifischen Nachweis von pathogenen Substanzen und/oder von Mikroorganismen oder zum intraoral orts- und stoffspezifischen Nachweis von Substanzen, die auf Munderkrankungen oder Heilungsprozessen hinweisen, geeignet sind.

Ebenso betrifft die Erfindung verformbare oder härtbare oder filmbildende dentale Abformmaterialien, die intraorale Stoffe ortspezifisch aufnehmen können, wobei diese aufgenommenen intraoralen Stoffe es dem Fachmann erlauben, durch Aufbringen diagnostisch wirksamer Zusatzstoffe auf die Trägermaterialien Testverfahren durchzuführen, die zum intraoral orts- und stoffspezifischen Nachweis pathogener Substanzen und/oder von Mikroorganismen oder die zum intraoral orts- und stoffspezifischen Nachweis von Substanzen, die auf Munderkrankungen oder Heilungsprozessen hinweisen, geeignet sind.

Der orts- und stoffspezifische Nachweis von Substanzen im Mundmilieu ist ein seit langem bearbeitetes Problem. Dem Fachmann sind Single-Site-Tests bekannt (z.B. EP-A-0 304 871), die alle darauf beruhen, dass von definierten Punkten im Mundraum, beispielsweise Zahnfleischtaschen, Zahnoberflächen oder Zahnwurzelkanälen einzelne Proben genommen werden. Die anschließende Analyse dieser Proben erfolgt je nach Fragestellung mit den unterschiedlichsten Methoden, wobei vier generelle Ansätze zu unterscheiden sind:
1. Die mikrobiologische Befunderhebung erfolgt häufig nach mehrtägiger Bebrütung der Proben in geeigneten Kulturmedien, weil die ursprünglich vorhandene Zahl von Mikroorganismen für eine direkte Befunderhebung nicht ausreichend ist. Nach Vermehrung der Mikroorganismen werden die Colony-Forming-Units (CFU) gezählt und auf die in der Probe befindlichen Zahl von Mikroorganismen geschlossen (Kneist, S.; Klein, C.; Rupf, S.; Eschrich, K. Quintessenz (1999) 50, 33-43). In diesen Testsystemen können sich die in der Probe befindlichen vitalen Mikroorganismen unter optimalen Bedingungen vermehren. Das Untersuchungsergebnis zeigt damit das maximal mögliche pathogene Potential des evaluierten Mikroorganismuses an, wenn sich der durch definierte Kulturmedien selektiv angezogene Mikroorganismus im Mundraum ähnlich ungehindert vermehren könnte.
   Bekanntlich liegen im Mundraum aber eben gerade nicht derartig optimale Wachstumsbedingungen vor, so dass das Testergebnis nur bedingt aussagekräftig ist.
   Darüber hinaus darf nicht übersehen werden, dass durch die Bebrütung der Proben eine Kultur pathogener Mikroorganismen angelegt wird, die mit den entsprechenden Vorsichtsmaßnahmen zur Risikominimierung in der Praxis behandelt werden müssen. Eine besondere Entsorgung ist erforderlich. Neben diesen Nachteilen sind die Bebrütungsverfahren zur mikrobiologischen Befunderhebung teuer und sehr zeitaufwendig.
2. Immunologische Methoden sind ein weiterer genereller Ansatz zur mikrobiologischen Befunderhebung in Single-Site-Tests. Hierbei werden monoklonale oder polyklonale Antikörper gegen Oberflächenstrukturen oder sezernierte Substanzen von Mikroorganismen eingesetzt. Darüber hinaus können mit entsprechenden Antikörpern beispielsweise auch Entzündungsvorgänge verfolgt werden. Beispielsweise sind hierfür WO-94/12877, US-5 665 559, WO-96/07103, WO-96/32647 zu nennen.
   Die immunologischen Methoden gemäß Absatz 2 sind im Vergleich zu den Bebrütungsverfahren gemäß Absatz 1 spezifischer, schneller und preisgünstiger, haben aber deutliche Schwächen in der Reproduzierbarkeit, die unter anderem durch die Probennahme bedingt werden. Beispielsweise befinden sich in einem Plaquebereich nicht nur vitale, sondern auch erhebliche Mengen abgestorbener Mikroorganismen. Je nach Probennahme kann das Verhältnis zwischen toten und vitalen Mikroorganismen unterschiedlich sein. Da die Antikörper nicht zwischen vitalen und toten Mikroorganismen unterscheiden können, ergibt sich eine unvorhersagbare Schwankungsbreite in der Ableitung des vorhandenen pathogenen Potentials der evaluierten Mikroorganismen (Aass, A.M.; Preus, H.R., Zambon, J.J., Gjermo, P. Scand J. Dent Res (1994) 102, 355 - 360).
3. Die Methode mit der höchsten Sensitivität beruht auf der Poly-Chain-Reaction-Technologie (PCR). Geringste Mengen Mikroorganismen können mit hoher Spezifität nachgewiesen werden. Allerdings ist die PCR-Technologie zeitaufwendig, komplex, kostenintensiv und in der Beherrschung nicht trivial (Rupf, S.; Kneist, S.; Merte,K.; Eschrich, K. Eur. J. Oral. Sci (1999) 107, 75 - 81).
4. Es wurden ferner einige Methoden beschrieben, die biochemische Marker nutzen, um Munderkrankungen zu diagnostizieren. Eine Übersicht bietet der Beitrag von J. Meyle, Deutsche Zahnärztliche Zeitschrift (1999) 54, 73-77).

Die Aussagekraft der einzelnen biochemischen Marker muß differenziert unter Berücksichtigung der klinischen Studien bewertet werden und bleibt dem Fachmann vorbehalten. Hervorzuheben ist, dass die Bestimmung der biochemischen Marker mittels Single-Site-Methoden erfolgt. Beispielsweise wird auf die Patentschrift WO-98/21583 hingewiesen. Die hier benötigten Hilfswerkzeuge zeichnen sich dadurch aus, dass sie die zu untersuchenden Proben binden (WO-91/14000, EP-A-0 304 871, US-A-5 725 373). Für jede Probenstelle muß jeweils ein Hilfswerkzeug eingesetzt und individuell analysiert werden.

Prinzipiell haben alle aus dem Stand der Technik bekannten Single-Site-Methoden den entscheidenden Nachteil, dass eine näherungsweise vollständige Situationsbeschreibung im Mundraum nur mit einer hohen Zahl von Einzelproben gewonnen werden kann. Zur Probennahme werden häufig Papierspitzen verwendet, die in Zahnfleischtaschen oder Wurzelkanäle eingeführt werden (US-A-5 725 373, EP-A-0 304 871).

Die WO 00/01350 beschreibt ein Modifikationsmaterial, welches eine Modifikationssubstanz enthält, welche die Fluoreszenz kranker Zellen oder von Bakterien verstärkt, indem sie die Produktion von Stoffwecheslzwischenprodukten des Stoffwechsels verstärkt, die fluoreszieren.

Die DE 17 45 810 befasst sich mit einem Verfahren zur Herstellung kautschukartiger Elastomere auf der Basis von Äthyleniminverbindungen durch Vernetzung.

Die DE 197 53 456 betrifft zweikomponentige, kationisch aushärtende Zubereitungen auf der Basis von Aziridinopolyethern und deren Verwendung. Die Zubereitungen können u.a. Modifikatoren, einschließlich Füllstoffen, Farbstoffen, Pigmenten, Thixotropiernitteln, Fließverbesserern, polymeren Eindickern, oberflächenaktiven Substanzen, Geruchsstoffen und Geschmacksstoffen enthalten.

In der WO 95/07286 werden bestimmte rekombinate DNA Moleküle beschrieben. Es wird ausgeführt, dass die enzymatische Aktivtät von Arg-gingipain-1 durch Glyzin und Glycin enthaltende Stoffe stimuliert werden kann.

Die EP 0 304 871 beschreibt ein Vorrichtung zur Detektion des Vorhandenseins von Krankheitskeimen im Mund. Die Vorrichtung umfasst ein absorbierendes Material, auf welches ein Enzym, ein Antigen oder eine Antikörper-reaktiven Substanz absorbiert ist.

Es ist bekannt, dass die Parodontitisaktivität von Zahnfleischtasche zu Zahnfleischtasche in einem Patienten sehr unterschiedlich sein kann, obwohl sich die Parodontitiserreger ubiquitär in den Zahrifteischtaschen befinden. Für eine Befunderhebung müssen deshalb weit mehr als 25 Einzelproben genommen und untersucht werden, ohne sicherstellen zu können, dass nicht der eine oder andere Parodontitisherd unberücksichtigt bleibt.

Hieraus wird prinzipiell einsichtig, dass punktuelle Bestandsaufnahmen nur unbefriedigende Situationsbeschreibungen des Mundraumes zulassen. Der hohe Zeit- und Kostenaufwand der Single-Site-Techniken ist damit nur bedingt zu rechtfertigen. Single-Site-Techniken haben sich daher In der Diagnostik des Mundraumes nicht In der breiten Anwendung durchgesetzt.

Es besteht daher selt langem ein dringendes Bedürfnis, ein einfaches und kostangünstiges Verfahren zur gleichzeitigen multiplen sowie orts- und stoffspezifischen intraoralen Befunderhebung im Mundraum zur Verfügung zu haben.

Aufgabe der vorliegenden Erfindung ist die Bereitstellung von Mitteln und Methoden zum intraoral orts- und stoffspezifischen sowie gleichzeitig multiplen Nachweis pathogener Substanzen und/oder von Mikroorganismen oder zum intraoral orts- und stoffspezifischen Nachweis von Substanzen, die auf Munderkrankungen oder Heilungsprozesse hinweisen.

Im Laufe der Beschreibung der Erfindung sind unter den nachzuweisenden pathogenen Substanzen und/oder Mikroorganismen oder Substanzen, die auf Munderkrankungen oder Heilungsprozessen hinweisen, beispielsweise nachfolgend aufgeführte zu verstehen:
1. Stoffwechselprodukte von Bakterien, Viren oder Pilzen, beispielsweise Antigene, Lipide, Proteine, Peptide, Polysaccharide, DNA, RNA, Zucker, Aminosäuren, Carbonsäuren, beispielsweise Milchsäure und Propionsäure, sowie andere niedermolekulare, anionische, kationische oder neutrale Substanzen sowie deren Kombinationen, die sich beispielsweise aus ionischen, polaren, unpolaren, hydrophoben, kovalenten oder adhäsiven Wechselwirkungen ergeben.
2. Oberflächenstrukturen von Bakterien, Viren oder Pilzen, bestehend beispielsweise aus Antigenen, Lipiden, Proteinen, Peptiden, Polysacchariden, DNA, RNA, Zuckern, Aminosäuren oder anderen niedermolekularen, anionischen, kationischen oder neutralen Substanzen sowie deren Kombinationen, die sich beispielsweise aus ionischen, polaren, unpolaren, hydrophoben, kovalenten oder adhäsiven Wechselwirkungen ergeben.
3. Humane bzw. tierische Substanzen, die als Antwort auf Infektionen durch Bakterien, Viren oder Pilze gebildet werden, bestehend beispielsweise aus Antikörpern, Antigenen, Lipiden, Proteinen, Peptiden, Polysacchariden, DNA, RNA, Zuckern, Aminosäuren oder anderen niedermolekularen, anionischen, kationischen oder neutralen Substanzen sowie deren Kombinationen, die sich beispielsweise aus ionischen, polaren, unpolaren, hydrophoben, kovalenten oder adhäsiven Wechselwirkungen ergeben.
4. Humane bzw. tierische Substanzen, die auf Munderkrankungen hinweisen, die nicht *a priori* auf eine Infektion durch Bakterien, Viren oder Pilze beruhen (beispielsweise Krebserkrankungen), bestehend beispielsweise aus Antikörpern, Antigenen, Lipiden, Proteinen, Peptiden, Polysacchariden, DNA, RNA, Zuckern, Aminosäuren oder anderen niedermolekularen, anionischen, kationischen oder neutralen Substanzen sowie deren Kombinationen, die sich beispielsweise aus ionischen, polaren, unpolaren, hydrophoben, kovalenten oder adhäsiven Wechselwirkungen ergeben.
5. Substanzen, die sich in Strukturen befinden, die als die Folge von oder die Voraussetzung für die Entstehung von Munderkrankungen, beispielsweise Plaque oder Biofilm, bekannt sind, bestehend beispielsweise aus Antikörpern, Antigenen, Lipiden, Proteinen, Peptiden, Polysacchariden, DNA, RNA, Zuckern, Aminosäuren oder anderen niedermolekularen, anionischen, kationischen oder neutralen Substanzen sowie deren Kombinationen, die sich beispielsweise aus ionischen, polaren, unpolaren, hydrophoben, kovalenten oder adhäsiven Wechselwirkungen ergeben.
6. Substanzen die auf laufende Heilungsprozesse hinweisen, die als die Folge von Munderkrankungen oder Verletzungen, beispielsweise Gewebe und/oder Knochenregeneration, bekannt sind, bestehend beispielsweise aus Antikörpern, Antigenen, Lipiden, Proteinen, Peptiden, Polysacchariden, DNA, RNA, Zuckern, Aminosäuren oder anderen niedermolekularen, anionischen, kationischen oder neutralen Substanzen sowie deren Kombinationen, die sich beispielsweise aus ionischen, polaren, unpolaren, hydrophoben, kovalenten oder adhäsiven Wechselwirkungen ergeben.

Die vorstehend aufgeführten Substanzen stehen exemplarisch für solche Substanzen, die alleine oder in Kombination für diagnostische Zwecke intraoraler Erkrankungen genutzt werden können und werden nachfolgend auch als Marker-Verbindungen bezeichnet.

Erfindungsgemäß wird die beschriebene Aufgabe gelöst durch verformbare, härtbare oder filmbildende dentale Abform- oder Filmmaterialien wobei diese Markerverbindungen binden bzw. aufnehmen, so dass die Diagnose am bzw. im Trägermaterial erfolgt, wie es in dem Ansprüchen beschrieben ist. Die Erfindung betrifft verformbares, härtbares oder filmbildendes Trägermaterial, welches dadurch gekennzeichnet ist, dass es für die orts- und stoffspezifische intraorale Diagnose diagnostisch nutzbare Zusatzstoffe enthält, die ohne Kultivierungsschritt zu einem diagnostischen Ergebnis führen. Die diagnostisch nutzbaren Zusatzstoffe dienen insbesondere zum intraoralen ortsspezifischen Nachweis von pathogenen Substanzen und/oder von Mikroorganismen oder zum intraoralen ortsspezifischen Nachweis von Substanzen, die auf Munderkrankungen oder Heilungsprozesse hinweisen. Die Zusatzstoffe können dabei in mikroverkapselter Form vorliegen. Die Trägermaterialien sollten mindestens soviel diagnostische Zusatzstoffe enthalten, dass ein diagnostisches Signal wahrgenommen werden kann.

Die Erfindung betrifft weiterhin ein Verfahren zur Herstellung von Abbildungen für intraorale orts- und stoffspezifische Diagnosezwecke, welche dadurch gekennzeichnet ist, dass diagnostisch nutzbare Zusatzstoffe auf verformbare, härtbare oder filmbildende dentale Abform-oder Filmmaterialien, die keine diagnostisch nutzbaren Zusatzstoffe enthalten, in einer solchen Menge aufgebracht werden, dass ein diagnostisches Signal wahrgenommen werden kann, wobei die Zusatzstoffe ohne Kultivierungsschritt zu einem diagnostischen Ergebnis führen, wie es in dem Ansprüchen beschrieben ist.

Die Erfindung kann verwendet werden in einem Verfahren für die gleichzeitige multiple sowie orts- und stoffspezifische intraorale Befunderhebung, umfassend die Schritte: Abdrucknahme mit verformbarem, härtbarem oder filmbildendem Trägermaterial, das diagnostisch wirksame Zusatzstoffe enthält, und gegebenenfalls Auftragen weiterer diagnostisch wirksamer Zusatzstoffe, oder Abdrucknahme mit verformbarem, härtbarem oder filmbildendem Trägermaterial, das keine diagnostisch wirksamen Zusatzstoffe enthält, und Auftragen diagnostisch wirksamer Zusatzstoffe.

Die erfindungsgemäß verwendbaren diagnostisch nutzbaren Zusatzstoffe sind zum Teil kommerziell erhältlich und können gegebenenfalls physikalisch, chemisch, biochemisch oder gentechnologisch verändert werden, wobei dies insbesondere für Enzyme und deren Substrate, für Antikörper und deren Antigene und für Oliginukleotide und Polynukleotide gilt.

Die diagnostisch nutzbaren Zusatzstoffe erlauben dem Fachmann die Durchführung diagnostischer Testverfahren, die zum intraoralen orts- und stoffspezifischen Nachweis pathogener Substanzen und/oder von Mikroorganismen oder die zum intraoral orts- und stoffspezifischen Nachweis von Substanzen, die auf Munderkrankungen oder Heilungsprozesse hinweisen, geeignet sind.

Zu den diagnostizierbaren Munderkrankungen gehören auch Karies, Early Onset Parodontitis, präpubertale Parodontitis, juvenile Parodontitis, schnell verlaufende progressive Parodontitis (RPP), adulte Parodontitis, refraktäre Parodontitis, Gingivitis, Halitosis, Infektionen mit Candida-albicans, Candida krusei, Candida glabrata, Candida lusitaniae, Candida dubliniensis, Krebs.

In Zahnfleischtaschen können sich Bakterien befinden, die den in Cystein oder Methionin befindlichen Schwefel in Form von flüchtigen Schwefelverbindungen wie Mercaptane oder Schwefelwasserstoff freisetzen. Daneben sind dissimilatorische sulfatreduzierende Bakterien bekannt, deren Schwefelwasserstoffbildung mit der Sulfatreduktion korreliert ist. Durch die Verwendung der erfindungsgemäßen Trägermaterialien und die Anwendung des erfindungsgemäßen Verfahrens lassen sich die Bildungsraten von Schwefelwasserstoff und Mercaptanen, bevorzugt Methylmercaptan in Zahnfleischtaschen messen. Darüber hinaus können die bakteriellen Enzymaktivitäten, bevorzugt Methionin-γ-lyase, besonders bevorzugt Cysteindesulfhydrase, die die Bildung der flüchtigen Schwefelverbindungen katalysieren, als Maß für die Halitosisaktivität von Zahnfleischtaschen gemessen werden. Darüber hinaus kann die Gegenwart der für die Freisetzung verantwortlichen Bakterien, bevorzugt Fusobakterien, Porphyromonas, Veillonella, Clostridium und Treponema, mit polyklonalen Antikörpern und deren Subklassen oder monoklonalen Antikörpern bestimmt werden.

Die verschiedenen Formen der Parodontitis sind kausal mit der Infektion durch Actinobacillus actinomycetemcomitans, Bacterioides forsythus, Campylobacter rectus, Capnocytophage ochracea, Capnocytophage gingivalis, Eikenella corrodens. Fusobacterium nucleatum. Porphyromonas asaccharolytikus, Porphyromonas gingivalis, Prevotella dentalis, Prevotella intermedia, Prevoltella nigrescens, Treponema denticola verbunden. Durch die Verwendung der erfindungsgemäßen Trägermaterialien und die Anwendung des erfindungsgemäßen Verfahrens lassen sich Gegenwart und Menge der Bakterien in der Sulkusflüssigkeit bestimmen. Hierfür eignen sich spezifische polyklonale Antikörper und deren Subklassen oder monoklonale Antikörper, die gegen Obeflächenantigene dieser Bakterien, beispielsweise Fimbriae, extrazelluläre Pollysaccharide, Adhesine gerichtet sind

Durch die Verwendung der erfindungsgemäßen dentalen Abform-oder Filmmaterialien und die Anwendung des erfindungsgemäßen Verfahrens können in der Sulkusflüssigkeit Enzymaktivitäten gemessen werden, die einen Hinweis auf die Gegenwart und metabolische Aktivität eines Bakteriums oder einer Gruppe der genannten Bakterien ergeben. Die Trypsin-ähnliche Protease-Aktivität, bevorzugt die Dipeptidylpeptidase-Aktivität, besonders bevorzugt Arg-Gingipain-Aktivität und Lys-Gingipain-Aktivität, wird diagnostisch genutzt. Zur Bestimmung der Arg-Gingipain-Aktivität können synthetische Peptide eingesetzt werden, die mindestens ein Arg-Rest (in P1-Position) neben der detektierbaren Abgangsgruppe enthalten. Zur Bestimmung der Lys-Gingipain-Aktivität können synthetische Peptide eingesetzt werden, die mindestens ein Lys-Rest (in P1-Position) neben der detektierbaren Abgangsgruppe enthalten. Neben p-Nitroanilin-Derivaten, beispielsweise Nα-Benzoyl-DL-arginin-p-nitroanilid, 2-Naphtylamin-Peptidderviaten, beispielsweise Nα-Benzoyl-DL-arginin-βnaphtylamid können 6-Aminoquinolin-Peptidderivate, Rhodamin-Peptidderivate sowie Cumarin-Peptidderivat, beispielsweise 7-Amido-4-methylcumarin, wie N-t-Boc-Val-Pro-Arg-7-Amido-4-methylcumarin und 7-Amino-4-chloromethylcumarin, wie N-t-Boc-Val-Pro-Arg-7-Amido-4-chloromethylcumarin als detektierbare Abgangsgruppen eingesetzt werden.

Durch die Verwendung der erfindungsgemäßen dentalen Abform-oder Filmmaterialien und die Anwendung des erfindungsgemäßen Verfahrens lassen sich mit polyklonalen Antikörpern und deren Subklassen oder monoklonalen Antikörpern die bakteriellen Substanzen diagnostizieren, die zur Induktion von Zytokinen führen. Bevorzugt werden Antikörper gegen Lipopolysaccharide, Lipoarabinomannan, Peptidoglycane, Teichonsäurederivate, extrazelluläre Polysaccharide und Lipid A.

Durch die Verwendung der erfindungsgemäßen dentalen Abform-oder Filmmaterialien und die Anwendung des erfindungsgemäßen Verfahrens kann die durch Parodontitiserreger induzierte Zytokininbildung mit polyklonalen Antikörpern und deren Subklassen oder monoklonalen Antikörpern diagnostiziert werden. Antikörper gegen die Interleukine IL-1, IL-2, IL-3, IL-4, IL-5, IL-6, IL-7, IL-8, Tumornecrosisfaktor TNFα, Interferone α,β,γ, Colony-forming Faktoren M-CSF, Wachstumsfaktoren EGF, TGFα und Chemokine MCP können eingesetzt werden.

Durch die Verwendung der erfindungsgemäßen dentalen Abform-oder Filmmaterialien, und die Anwendung des erfindungsgemäßen Verfahrens kann die Zerstörung parodontalen Gewebes über die Enzymaktivitäten von alkalischer Phosphatase, Arylsulfatase, Aspartataminatransferase, β-Glucuronidase, Cathepsine (G.B.D). Elastase, Hyaluronidase, Lactatdehydrogenase, Lysozym, Matrixmetalloproteinasen (Kollagenasen, Gelatinasen), Tissue Inhibitors Metalloproteinases (TIMP), Stromelysin, Lactoferrin, Tryptase und Myeloperoxidase diagnostiziert werden.

Durch die Verwendung der erfindungsgemäßen dentalen Abform-oder Filmmaterialien und die Anwendung des erfindungsgemäßen Verfahrens können mit polyklonalen Antikörpern und deren Subklassen oder monoklonalen Antikörpern die molekularen Marker für Gingivitis diagnostiziert werden. Zu diesen gehören Zytokine, beispielsweise Interleukine IL-1, IL-2, IL-4, IL-6, TNFα und Arachidonsäurederviate, beispielsweise Prostaglandin E₂.

Karies ist kausal mit der Infektion durch Streptococcus salivarius salivarius, Streptococcus vestibularis, Streptococcus thermophilus, Streptococcus mutans, Streptococcus rattus, Streptococcus sobrinus, Streptococcus cricetus, Streptococcus downei, Streptococcus macacae, Streptococcus ferus, Streptococcus milleri, Streptococcus anginosus, Streptococcus constellatus, Streptococcus intermedius, Strepto-coccus mitis, Streptococcus oralis, Streptococcus sanguis, Streptococcus gordonii, Strepto-coccus parasanguis, Streptococcus crista, Streptococcus mitior, Lactobacillus acidophilus, Lactobacillus alimentarius, Lactobacillus brevis, Lactobacillus buchneri, Lactobacillus casei, Lactobacillus paracasei ss paracasei, Lactobacillus paracasei ss rhamnosus, Lactobacillus paracasei ss tolerans, Lactobacillus delbrueckii, Lactobacillus delbrueckii ss lactis, Lacto-bacillus delbrueckii ss delbrueckii, Lactobacillus delbrueckii ss bulgaricus, Lactobacillus endocarditis, Lactobacillus fermentum, Lactobacillus gasseri, Lactobacillus pseudoplantarum, Lactobacillus rhamnosus, Lactobacillus salivarius, Actinomyces israelii. Actinomyces odontolyticus, Actinomyces actinomycetemcomitans, Eikenella, Branhamella catarrhalis, Veillonella alcalescens, veillonella parvula, Actinomyces naestundii, Rothia dentocariosa, verbunden. Durch die Verwendung der erfindungsgemäßen Trägermaterialien und die Anwendung des erfindungsgemäßen Verfahrens können mit polyklonalen Antikörpern und deren Subklassen oder monoklonalen Antikörpern, die gegen die verschiedenen Oberflächenantigene dieser Bakterien, beispielsweise Proteine. Lipopolysaccharide, Glycoproteine, Fimbriae, extrazelluläre Pollysaccharide, Adhesine, Lipoteichonsäurederivate, Glucan-Bindungsproteine, Collagen-Blndungsproteine gerichtet sind, Gegenwart und Menge der kariogenen Bakterien diagnostiziert werden.

Durch die Verwendung der erfindungsgemäßen dentalen Abform-oder Filmmaterialien und die Anwendung des erfindungsgemäßen Verfahrens können extrazelluläre Enzymaktivitäten kariogener Bakterien diagnostiziert werden, beispielsweise Proteasen, bevorzugt Glucosyltransferasen, Glucanase, Fructosyltransferase, Fructanase.

Durch die Verwendung der erfindungsgemäßen dentalen Abform-oder Filmmaterialien und die Anwendung des erfindungsgemäßen Verfahrens können Stoffwechselprodukte kariogener Bakterien diagnostiziert werden, beispielswesie Buttersäure, Ameisensäure, bevorzugt Essigsäure, Propionsäure, besonders bevorzugt Milchsäure. Die mit der Säurefreisetzung einhergehende Versauerung des umgebenden Milieus kann darüber hinaus mit pH-Indikatoren, beispielsweise mit Bromphenolblau, Kongorot, Bromkresolblau, bevorzugt Rhodolderivate, besonders bevorzugt Oregon Green-Derivate, nachgewiesen werden. Als Folge der Versauerung des pHs im umgebenden Milieu, wie Plaque, werden aus der Zahnhartsubstanz Calciumionen herausgelöst. Durch die Verwendung der erfindungsgemäßen Trägermaterialien und die Anwendung des erfindungsgemäßen Verfahrens kann dieser Prozess mit Calciumindikatoren, beispielsweise Calcium Crimson, bevorzugt Calcium Green, Calcium Orange, besonders bevorzugt Calcium Oregon Green 488 BAPTA, diagnostiziert werden.

Durch die Verwendung der erfindungsgemäßen dentalen Abform-oder Filmmaterialien und die Anwendung des erfindungsgemäßen Verfahrens kann die Zunahme oder die Abnahme der oben genannten Markerverbindungen als Maß für den Heilungsprozess herangezogen werden.

Die Liste der Markerverbindungen ist beispielhaft und nicht limitierend für die Erfindung.

Überraschend ist, dass trotz der ablaufenden dynamischen Prozesse in der Mundhöhle, die einem ständigen Flüssigkeitsaustausch durch die Sekrete der Speicheldrüsen und der Sulkusflüssigkeit unterliegt, ausreichend hohe Konzentrationen von Marker-Verbindungen auf den Oberflächen der erfindungsgemäßen Trägermaterialien oder in den Trägermaterialien erhalten werden, die es gestatten, eine sichere Diagnose auch im Rahmen von Routinebehandlungen zu realisieren.

Vorteilhaft ist es dass durch die Verwendung der erfindungsgemäßen dentalen Abform-oder Filmmaterialien oder durch den Einsatz des erfindungsgemäßen Verfahrens eine nahezu komplette Situationsbeschreibung des Mundraumes, unter Verzicht einer großen Anzahl von Einzelproben, sowie eine Archivierung des momentanen Krankheitsbildes möglich ist. Hierbei ist besonders die Verwendung von additionsvernetzenden Silikonabformmaterialien von Interesse, da die Abdrücke praktisch unbegrenzt haltbar sind. Gegebenenfalls können zur Archivierung des momentanen Krankheitsbildes die Abdrücke auch mittels Photographie, digitalen Kameras, UV-VIS bzw. Fluoreszenz-Scanner erfasst und mittels Bilddokumentationssoftware ausgewertet werden.

Vorteilhaft ist es außerdem, dass durch die Verwendung der erfindungsgemäßen dentalen Abform-oder Filmmaterialien und die Anwendung des erfindungsgemäßen Verfahrens eine nahezu komplette Situationsbeschreibung der einzelnen Zähne, unter Verzicht einer großen Anzahl von Einzelproben, sowie eine Archivierung des momentanen Krankheitsbildes möglich ist. Neben okklusalen Kauflächen und vestibulären, lingualen, koronalen, apikalen, zervikalen, gingivalen, inzistalen Bereichen eines Zahnes werden durch die Zeichnungsschärfen der Trägermaterialien auch die interproximalen Bereiche zwischen den Zähnen erfaßt.

Vorteilhaft ist es auch, dass durch die Anwendung der erfindungsgemäßen dentalen Abform-oder Filmmaterialien oder des erfindungsgemäßen Verfahrens gegebenenfalls Flüssigkeit aus den Zahnfleischtaschen gesammelt und der orts- und substanzspezifischen Diagnose zugeführt werden kann. Eine nahezu komplette Situationsbeschreibung der einzelnen parodontalen Taschen, unter Verzicht einer großen Anzahl von Einzelproben, sowie eine Archivierung des momentanen Krankheitsbildes ist damit möglich.

Vorteilhaft ist überdies, dass die orts- und stoffspezifische intraorale Diagnose derart erfolgt, dass die diagnostisch nutzbaren Zusatzstoffen den Patienten nicht belasten, weil die Abgabe der diagnostisch nutzbaren Zusätze vermieden wird. Die diagnostisch nutzbaren Zusatzstoffe sind damit keine Modifikationssubstanzen, die die intraoral ablaufender Prozesse modifizieren. Eine wiederholte Anwendung der orts- und stoffspezifischen intraoralen Diagnose zum Verfolgen des Behandlungsverlaufes wird damit ermöglicht.

Vorteilhaft ist es ferner, dass durch die Anwendung der erfindungsgemäßen dentalen Abform-oder Filmmaterialien oder des erfindungsgemäßen Verfahrens das zeitaufwendige Kultivieren oder Inkubieren pathogener Mikroorganismen entfällt und somit auch das mit der Vermehrung pathogener Keime verbundene Risiko minimiert wird. Ein besonders großer Vorteil der erfindungsgemäßen Methode besteht gerade darin, dass die Nachweise auch dann gelingen, wenn die Konzentrationen der nachzuweisenden Substanzen im Abbildungsmaterial sehr gering sind.

Vorteilhaft ist zusätzlich, dass das Diagnoseergebnis vom Abdruck gegebenenfalls auf ein Positivabdruck übertragbar ist. Dies ist beispielsweise mit Gips, Hydrogelen, Modellsilikonen oder ähnlichen Massen möglich. Die Zuordnung der Diagnosesignale im Abdruck zu den einzelnen Zähnen wird damit erleichtert.

Mit den erfindungsgemäßen dentalen Abform-oder Filmmaterialien gelingt auch der direkte orts- und stoffspezifische Nachweis von Mikroorganismen auf den Zähnen, ohne die auf den Trägermaterial haftenden Mikroorganismen kultivieren oder inkubieren zu müssen. Somit entfällt beispielsweise auch der Zusatz von Nährstoffen zum Trägermaterial, wie dies in der US-A-4 976 951 beschrieben ist.

Ebenso vorteilhaft ist die Einfachheit der beschriebenen Verfahren, die bei vielen Erkrankungen eine problemlose Früherkennung bzw. Frühdiagnose mit geringem Aufwand und ohne wesentliche Mehrkosten für den Behandler und den Patienten gestattet.

Als dentales Abform- oder Filmmaterial kommen beispielsweise dentale Abformmassen oder Filme, jeweils auf Silikon-, Polyether-Silikon-, Polyether-, Alginat- oder Hydrokolloidbasis in Frage. Für manche Anwendungsbereiche, wie der Kariesdiagnose werden Alginate, bevorzugt ohne Zusatz von Phosphaten oder Pyrophosphaten verwendet. Ebenso geeignet als dentales Abform- oder Filmmaterial sind alle anderen bekannten Kunststoffe, beispielsweise Polyethylene, Polypropylene, Poly(meth)acrylate, Polyurethane, Polycarbonate, Polysulfid, Polyvinylchloride oder Kautschuk. Darüber hinaus sind Hydrogele, beispielsweise auf Polyvinylpyrrolidon- oder Polyvinylalkoholbasis, als dentales Abform- oder Filmmaterial geeignet. Gleichfalls geeignet für die Durchführung der erfindungsgemäßen Verfahren sind dentale Gipszubereitungen, nicht auszuhärtende plastische Massen, wie Knetmassen oder Festkörperdispersionen in Flüssigkeiten, beispielsweise Pasten und ähnliche Massen aus Silikon, Wachsen, Gelatine, Stärke, Fette und den oben genannten dentalen Abform-oder Filmmaterialien.

Die Basis vieler Abdruckmassen bilden additionsvernetzende oder kondensationsvernetzende Silikone, Polyether-Silikone oder Polyether. Diese Materialien sind im Stand der Technik ausführlich beschrieben worden, so dass es sich erübrigt, hier näher darauf einzugehen. Additions- oder kondensationsvernetzende Silikone sind beispielsweise in der US-A-3 897 376, in der EP-B-0 231 420 sowie in der dort auf Seite 3 erwähnten US-A-4 035 453, weiterhin in der EP-A-0 480 238 (siehe insbesondere Seite 2, Zeilen 3 - 26) und in der EP-B-0 268 347 beschrieben. Die Offenbarung dieser Schriften soll hier durch Inbezugnahme mitumfasst sein. Polyether-Silikone sind unter anderem beispielsweise in der DE-A-37 41 575 sowie in der DE-A-38 38 587 beschrieben, deren Offenbarung hier ebenfalls mitumfasst sein soll. Polyether sind beispielsweise in der DE-B-17 45 810, DE-A-43 06 997, DE-A-40 93 555, DE-C-25 15 593, DE-A-197 19 438 und US-A-34 53 242 beschrieben, deren Offenbarung hier gleichfalls mitumfasst sein soll. Bevorzugt werden Abformmassen auf N-Alkylaziridinopolyetherbasis.

Insbesondere sind dentalen Abform-oder Filmmaterialien auf Polyetherbasis geeignet. Hierbei umfassen die Massen beispielsweise folgende Bestandteile:
(A) 30 bis 96,9999, bevorzugt 40 bis 88,99, besonders bevorzugt 45 bis 80,49 Gew.-% mindestens eines N-Alkylaziridinopolyethers mit einer Molmasse im Bereich von 1.000 bis 20.000 g/Mol und einer Aziridinoäquivalentmasse im Bereich von 500 bis 8.000 g/Äquivalent,
(B) 1 bis 10, bevorzugt 1 bis 5, besonders bevorzugt 1,5 bis 3 Gew.-% Startersubstanzen, die geeignet sind, die Aushärtung der N-Alkylaziridinopolyether zu bewirken,
(C) 1 bis 50, bevorzugt 5 bis 45, besonders bevorzugt 8 bis 43 Gew.-% organische Verdünnungsmittel,
(D) 1 bis 50, bevorzugt 5 bis 40, besonders bevorzugt 10 bis 30 Gew.-% Modifikatoren, einschließlich Füllstoffen, Farbstoffe, Pigmente, Thixotropiemittel, Fließverbesserer, polymere Eindicker, oberflächenaktiven Substanzen, Geruchsstoffe und Geschmacksstoffe,
(E) 0,0001 bis 10 Gew.-%, bevorzugt 0,01 bis 1 Gew.-% diagnostische Zusatzstoffe.

Bestandteil (A) umfasst N-Alkylaziridinopolyether, wobei die Polyether-Grundkörper Homopolymere aus Ethylenoxid, Propylenoxid oder Tetrahydrofuran, statistische Co- und Terpolymere der genannten Monomeren und bzw. oder Blockcopolymere aus Ethylenoxid und Propylenoxid sein können. Für die Verwendung in zweikomponentigen Abformmassen sind solche Startersubstanzen gemäß Bestandteil (B) geeignet, die eine Aushärtung der gemischten Zubereitung in einem Zeitraum von 1 bis 20 Minuten zu einem elastischen Festkörper ermöglichen, wobei dieser Festkörper die Anforderungen an eine elastische Abformmasse gemäß DIN / EN 2482 erfüllt und eine Shore A-Härte (DIN 53505) von mindestens 20 nach 24 Stunden Lagerzeit besitzt.

Als Starter der Katalysatorkomponente können viele der bekannten Starter eingesetzt werden. Zweckmäßigerweise verwendet man solche Starter bzw. Startersysteme, die eine einfache Einstellung des Aushärtungsverlaufs zulassen, keine Nebenwirkungen erzeugen und die reproduzierbare Erreichung der erforderlichen Niveaus der mechanischen Eigenschaften ermöglichen.

In der DE-C-914 325 wird die Verwendung von Oxonium-, Ammonium- und Sulfoniumsalzen als Startersubstanzen vorgeschlagen.

Eine zusammenfassende Darstellung der für die Aushärtung von N-Alkylaziridinoverbindungen verwendeten Startersubstanzen ist in O. C. DERMER, G. E. HAM, "Ethylenimine and other Aziridines" Academic Press (1969) enthalten.

Als prinzipiell geeignete Polymerisationsauslöser haben sich demnach eine große Anzahl von Verbindungsklassen und Verbindungen erwiesen. In der praktischen Anwendung der kationischen Polymerisation von Aziridinopolyethern ist es aber sehr schwierig, den gewünschten Abbindeverlauf mit ausreichend langer Verarbeitungszeit und schneller Endaushärtung einzustellen. Dieses Ziel kann durch die Verwendung von speziellen Trisalkylsulfoniumsalzen erreicht werden, wie sie beispielsweise in der EP-A-0 110 429 beschrieben sind.

Unter Verwendung von speziellen Trisalkylsulfoniumsalzen sind die Kriterien der die Härtungsgeschwindigkeit und der Eigenschaften des elastischen Festkörpers prinzipiell erreichbar.

In der Patentanmeldung DE-A-100 18 918 werden Starter beschrieben, die der Katalysatorkomponente einen lediglich geringen Säuregrad verleihen und die eine gut einstellbare, relativ lange Verarbeitungszeit nach erfolgter Mischung von Basiskomponente und Katalysatorkomponente ermöglichen.

Startersysteme dieses Typs sind geeignet, die Basispasten in der notwendigen Geschwindigkeit auszuhärten. Durch ihre Verwendung sind die gewünschten Eigenschaften des elastischen Festkörpers erreichbar.

Die Patentanmeldung DE-A-199 42 459 beschreibt Elastomermassen mit verbesserter Katalysatorkomponente, die sich durch eine erhöhte Dehnbarkeit auszeichnen. Gemäß dieser Erfindung werden Borsäurekomplexe als Starter eingesetzt. Diese Starter haben sich für die Aushärtung der N-Alkylaziridinopolyether besonders bewährt.

Als organisches Verdünnungsmittel, entsprechend Bestandteil (C), werden Polyetherpolyole, wie Polypropylenglykole oder Mischpolyetherole mit Tetrahydrofuran- und/oder Ethylenoxid- und/oder Propylenoxid-Einheiten, Polyesterpolyole, wie Polycaprolactondiole und Polycaprolactontriole, Polycarbonatdiole, aliphatische Ester, Öle, Fette, Wachse, aliphatische Kohlenwasserstoffe, araliphatische Kohlenwasserstoffe sowie ein- oder mehrfunktionelle Ester von mehrwertigen Säuren, wie Phthalsäure oder Zitronensäure oder Ester oder Amide von Alkylsulfonsäuren und Arylsulfonsäuren, verwendet.

Die Modifikatoren gemäß Bestandteil (D) sind meist feinteilige Füllstoffe, wie Alumosilikate, Fällungskieselsäuren, Quarzmehl, Wollastonit, Glimmermehl und Diatomeenerde, sowie Farbstoffe und Pigmente, deren Zusatz eine bessere Beurteilung der Mischgüte ermöglicht und die Verwechslungsgefahr vermindert, Thixotropiemittel, wie feindisperse Kieselsäuren und andere das Fließverhalten beeinflussende Zusätze, wie polymere Eindicker, weiterhin oberflächenaktive Substanzen zur Einstellung des Anfließverhaltens sowie Geruchsstoffe und Geschmacksstoffe.

Ein weiteres mögliches, dentales Abform-oder Filmmaterial kann auch eine polymerisierbare Flüssigkeit oder eine Lösung einer polymeren Substanz sein, die auf die zu untersuchenden Stellen aufgesprüht oder aufgetragen, beispielsweise aufgepinselt wird. Typischerweise handelt es sich hierbei um Lacke auf Nitrocellulosebasis mit einem flüchtigen Lösungsmittel sowie gegebenenfalls weiteren Hilfsstoffen, die zu einer festen Schicht aushärten, die nach Aufnahme der Markerverbindung vom Substrat abgezogen werden kann. Verwendbar sind allgemein alle Polymeren, die in geeigneten leicht flüchtigen Lösungsmitteln aufgenommen werden können. Bekannt ist beispielsweise auch die Verwendung von Polyurethanen in Aceton. Geeignete filmbildende Systeme sind aus der Farben- und Lackchemie hinreichend bekannt.

Das erfindungsgemäße dentales Abform-oder Filmmaterial kann zunächst die zu untersuchende Markerverbindung intraoral ortspezifisch aufnehmen. Die Markerverbindung wird in einer anschließenden Prozedur auf bzw. im dentalen Abform- oder Filmmaterial orts- und stoffspezifisch nachgewiesen, quantifiziert bzw. diagnostisch evaluiert, wobei die Markerverbindung auch erst als Folge einer katalytischen, chemischen, biochemischen Reaktion gebildet werden kann. Die zu analysierende Markerverbindung kann beispielsweise über ionische, polare, unpolare oder hydrophobe Wechselwirkungen auf bzw. im dentalen Abform- oder Filmmaterial örtlich fixiert werden. Die Ausbildung von Mikrostrukturen und/oder Mikroräumen in den dentalen Abform- oder Filmmaterial beispielsweise in Form von Schäumen kann die Aufnahme und Fixierung der zu untersuchenden Markerverbindungen unterstützen.

Das dentales Abform- oder Filmmaterial enthält in einer bevorzugten Ausführungsform mindestens eine Komponente oder aber zur Vereinfachung der diagnostischen Prozedur alle benötigten Komponenten des diagnostischen Testsystems. Diese diagnostischen Zusätze können beispielsweise über ionische, polare, unpolare oder hydrophobe Wechselwirkungen auf bzw. im dentalen abform- oder filmmaterial örtlich fixiert werden. Eine örtliche Fixierung von diagnostischen Zusätzen ist auch dadurch möglich, dass die diagnostischen Zusätze zuerst an das hochmolekulare dentale Abform- oder Filmmaterial fixiert und anschließend in die dentale Abform- oder Filmmaterial masse eingeknetet werden. Hierdurch wird die Diffusionsbewegung der diagnostischen Zusätze im dentalen Abform- oder Filmmaterial kontrolliert. Die Ausbildung von Mikrostrukturen und/oder Mikroräumen in den dentale Abform-oder Filmmaterialien beispielsweise in Form von Schäumen kann die Aufnahme und Fixierung der Komponenten unterstützen. Die Komponenten können in den erfindungsgemäßen dentalen Abform-oder Filmmaterialien frei verfügbar oder in einer anderen Phase vorliegen.

Die erfindungsgemäßen dentalen Abform-oder Filmmaterialien enthalten 0,0001 bis 10 Gew.-%, bevorzugt 0,01 bis 1 Gew.-% diagnostische Zusätze, jedoch mindestens soviel Zusätze, dass die gewünschte Wirkung wahrgenommen werden kann. Bei der Anwendung des erfindungsgemäßen Verfahrens müssen diagnostische Zusätze in einer solchen Menge auf die dentalen Abform-oder Filmmaterialien aufgebracht werden, dass die gewünschte Wirkung wahrgenommen werden kann.

Erwünschte Wirkungen können alle wahrnehmbaren Signale sein. Hierunter mit eingeschlossen sind beispielsweise Farbsignale, beispielsweise fluoreszierende, UV-, VIS-, phosphoreszierende oder lumineszierende Signale, die gegebenenfalls mit speziellen Geräten detektiert werden müssen. Ebenso können Signale durch Anwendung der erfindungsgemäßen Verfahren erzeugt werden, die durch Thermographie, Spektroskopie. Chromatographie oder auch durch Analyse der Topographieänderung der dentalen Abform-oder Filmmaterialien wahrgenommen werden können.

Diagnostische Zusätze sind beispielsweise, ohne dass die folgende Aufzählung limitierend für die vorliegende Erfindung zu verstehen wäre:
- Farbstoffindikatoren, beispielsweise pH-Indikatoren, wie Bromphenolblau, Kongorot, Bromkresolgrün, Oregon Green-Derivate, Rhodol-Derivate, RedoxIndikatoren, wie Methylenblau, 5-Cyano-2,3-ditolyltetrazoliumchlorid (CTC), 2-(4-Iodophenyl)-3-(4-nitrophenyl)-5-phenyl-2H-tetrazoliumchlorid (INT). 8-Dimethylamino-2,3-benozophenoxazin (Meldola's Blau), 1-Methoxyphenazinmethosulphat (MPMS), 5-(3-Carboxymethoxyphenyl)-2-(4,5-dimethylthiazolyl)-3-(4-sulphophenyl)tetrazolium (MTS), 3-(4,5-Dimethylthiazol-2-yl)-2,5-diphenyltetrazoliumbromid (MTT), 3,3'-(3,3'-Dimethoxy-4,4'-biphenylene)-bis[2-(4-nitrophenyl-5-phenyl)]-2H-tetrazoliumchlorid (NBT), Nitrotetrazoliumviolett (NTV), Phenazinmethosulphat (PMS), Natrium-3'-[1-[(phenylamino) carbonyl]-3,4-tetrazolium]bis(4-methoxy-6-nitro)benzolsulfonsäure (XTT), Phenazinethosulfat (PES), WST-1)
- Fluoreszenzindikatoren, beispielsweise Oregon Green 488 BAPTA, Calcium Green, Calcium Orange, Calcium Crimson,
- Chemolumineszenz-Indikatoren,
- Vitalitätsindikatoren, beispielsweise 5-Bromo-2' deoxyuridine,
- Andere Farbstoffindikatoren, beispielsweise p-Nitroaniline-Derivate, 2-Naphthylamin-Derivate, 7-Amino-4-methylcoumarin-Derivate, 7-Amino-4-chloromethylcoumarin-Derivate, 6-Aminoquinolin-Derivate, Rhodamin-Derivate, 5,5'-Dithiobis-(2-nitrobenzoesäure), Monobrombiman-Derivate, Tetramethylrhodamin-Derivate, Eosin-Derivate, Erythrosin-Derivate, Texas Red-Derivate, Coumarin-Derivate, Pyridyloxauzol-Derivate, Benzofurazan-Derivate, Naphtalin-Derivate, Didansyl-Cysteine, Dansyl-Derivate, Aziridin-Derivate, Pyren-Derivate, Coomassie Blau)

Darüber hinaus können die Indikatorsubstanzen beispielsweise kovalent an Enzymen, Proteinen, Glycoproteinen, Lipopolysacchariden, Polysacchariden, polyklonalen und monoklonalen Antikörpern, DNA, RNA Zellorganellen oder Mikroorganismen gebunden sein.

Unter diagnostischen Zusätzen werden auch Antikörper, die gegen Markerverbindungen gerichtet sind, sowie polyklonale Antikörper und deren Subklassen, sowie monoklonale Antikörper verstanden. Darüber hinaus können die Antikörper beispielsweise kovalent an Enzymen, Proteinen, Glycoproteinen, Lipopolysacchariden, Polysacchariden, DNA, RNA, Zellorganellen, Mikroorganismen oder anderen Trägermaterialien gebunden sein.

Diagnostische Zusätze können Enzyme folgender Klassen sein, wobei die folgende Aufzählung beispielsweise und nicht limitierend für die Erfindung ist:
- Oxidoreductasen und deren Unterklassen, beispielsweise Dehydrogenasen, wie Lactatdehydrogenase, Oxidasen, Peroxidasen, Reductasen, Monooxygenasen, Dioxygenasen;
- Transferasen und deren Unterklassen, beispielsweise C₁-Transferasen, Glycosyl-Transferasen, wie Glusoyltransferasen, Fructosyltransferasen, Aminotransferasen, Phospho-Transferasen;
- Hydrolasen und deren Unterklassen, beispielsweise Esterasen, Glycosidasen, wie Glucanase, Fructanase, Peptidasen, beispielsweise Dipeptidylpeptidasen Arg-Gingipain, Lys-Gingipain, Collagenasen, Gelatinasen, Cathepsine, Elastase, Amidasen,
- Lyasen und deren Unterklassen, beispielsweise C-C-Lyasen, C-O-Lyasen, C-N-Lyasen, C-S-Lyasen;
- Isomerasen und deren Unterklassen, beispielsweise Epimerasen, cis-translosmerasen, intramolekulare Transferasen;
- Ligasen und deren Unterklassen, beispielsweise C-C-Ligasen, C-O-Ligasen, C-N-Ligasen, C-S-Ligasen.

Man kennt heute über 2000 verschiedene Enzyme. Zu ihrer Klassifizierung wurde ein System entwickelt, das Wirkungs- und Substratspezifität berücksichtigt. Daraus ergibt sich, dass zu jedem Enzym spezifische Substrate und/oder Coenzyme (NAD(P), NAD(P)H, FAD, FMN, Liponamid, Ubichinon, Häm, ATP, ADP, AMP, GTP, GDP, GMP, UTP, UDP, UMP, CTP, CDP, CMP, Coenzym A, Thiamindiphosphat, Pyridoxalphosphat, Biotin, Tetrahydrofolat gehören. Diese spezifischen Substrate und/oder Coenzyme müssen als diagnostischer Zusatz vorhanden sein, wenn beispielsweise ein oder mehrere Enzyme als Markersubstanz dienen. Umgekehrt gilt natürlich, dass spezifische Enzyme als diagnostische Zusätze verwendet werden können, wenn spezifische Substrate beispielsweise Zuckerphosphate, Milchsäure/Lactat, Pyruvat, Essigsäure/Acetat, Propionsäure/Propionat, Ameisensäure/Formiat, Peptide, synthetische Peptide als Markersubstanzen dienen.

Darüber hinaus können die Enzyme kovalent an das dentale Abform- oder Filmmaterial gebunden sein.

Diagnostische Zusätze können auch solche Substanzen sein, die begleitend vorliegen müssen, um die Markersubstanzen diagnostizieren zu können. Solche Substanzen umfassen:
- Puffersubstanzen, beispielsweise Natriumphosphat, Natriumhydrogenphosphat, Natriumdihydrogenphophat, Kaliumphosphat, Kaliumhydrogenphosphat, Kaliumdihydrogenphosphat, Natriumpyrrophosphat, Natriumcarbonat, Kaliumcarbonat, Natriumhydrogencarbonat, Kaliumhydrogencarbonat, Natriumtetraborat, Essigsäure/Acetat, Citronensäure/Citrat, Diethylbarbitursäure, Tris(hydroxymethyl)aminomethan (TRIS), Glycin, Glycylglycin, N-(2-Acetamido)-2-aminoethansulfonsäure (ACES), N-(2-Acetamido)iminodiacetat (ADA), N,N-Bis(2-hydroxyethyl)-2-aminoethansulfonsäure (BES), N,N-Bis(2-hydroxyethyl)glycin (BICINE), 2,2-Bis-(hydroxyethyl)-iminotris(hydroxymethyl)methan (BIS-TRIS), 2-(Cyclohexylamino)ethansulfonsäure (CHES), 2-[4-(2-Hydroxyethyl-1-piperazin)]ethansulfonsäure (HEPES), 3-[4-(2-Hydroxyethyl-1-piperazinyl)]propansulfonsäure (HEPPS), 2-Morpholinoethansulfonsäure (MES), 3-Morpholinopropansulfonsäure (MOPS), Piperazin-1,4-bis(2-ethansulfonsäure) (PIPES), N-[Tris(hydroxymethyl)-methyl]-2-aminoethansulfonsäure (TES), N-[Tris(hydroxymethyl)-methyl]-glycin (TRICINE);
- Säuren, beispielsweise Schwefelsäure, schweflige Säure, Phosphorsäure, Salzsäure, Essigsäure, Salpetersäure;
- Basen, beispielsweise Natriumhydroxid, Kaliumhydroxid, Lithiumhydroxid, Ammoniak, Calciumhydroxid, Magnesiumoxid;
- Lösungsmittel, beispielsweise Wasser, Methanol, Ethanol, Isopropanol, Propanol, Glycerin, Dimethylsulfoxid, Tetrahydrofuran, Aceton, Butanon, Cyclohexan, Toluol, Methylenchlorid, Chloroform, Alkane, Essigsäurethylester;
- Salze, beispielsweise Magnesiumchlorid, Magnesiumsulfat, Magnesiumnitrat, Calciumchlorid, Calciumsulfat, Calciumnitrat, Eisen(III)chlorid, Eisen(II)chlorid, Zinkchlorid, Zinksulfat, Nickelchlorid, Manganchlorid, Ammoniumsulfat, Natriumsulfat, Natriumchlorid, Kaliumchlorid, Natriumphosphate, Kaliumphosphate;
- andere Substanzen, beispielsweise Glutathion, Rinderserumalbumin, Saccharose, Glucose, Fructose, Trehalose, Polyethylenglycol, Polyvinylpyrrolidon, Wasserstoffperoxid.

In einer speziellen Ausführungsform der Erfindung können die diagnostischen Zusätze in mikroverkapselter Form vorliegen. In einer Mikrokapsel kann eine Vielzahl von Molekülen diagnostischer Zusatzstoffe eingeschlossen sein. Von besonderem Vorteil ist bei der Verwendung von mikroverkapselten diagnostischen Substanzen der auftretende Potenzierungseffekt.

Ganz allgemein können bei der Verwendung mehrkomponentiger Diagnosesysteme gemäß der Erfindung, also von Systemen, bei denen die notwendigen Bestandteile zum Nachweis in mehreren Komponenten gelagert werden, die einzelnen Komponenten getrennt voneinander, jedoch jeweils eingeschlossen in Mikrokapseln, oder auch teilweise mikroverkapselt und teilweise frei vorliegen. Selbstverständlich ist es auch möglich, bei mehr als zweikomponentigen Diagnosesystemen, mindestens zwei Komponenten jeweils mikroverkapselt und mindestens eine andere Komponente frei im Trägermaterial vorrätig zu halten. Essentiell ist jeweils nur, dass eine Reaktion der diagnostischen Zusätze zum gewünschten Endprodukt durch das getrennte Vorhalten der einzelnen Komponenten solange unterbunden wird, bis ein Reaktionspartner durch eine Zerstörung der Mikrokapselwand freigesetzt wird. Da Abformmaterialien üblicherweise zweikomponentig angeboten werden, kann es vorteilhaft sein, verschiedene Komponenten der Wirkstoffe in verschiedenen Komponenten der Abformmassen, namentlich der Basis- und der Katalysatorpaste, mikroverkapselt oder frei vorzuhalten.

Bei der Auswahl von geeigneten dentalen Abform-oder Filmmaterialien ist allgemein darauf zu achten, dass diese mit den diagnostischen Substanzen kompatibel sind. Beispielsweise sollte bei der Verwendung von Fluoreszenzfarbstoffen die dentalen Abform-oder Filmmaterialien selbstverständlich keine Bestandteile enthalten, die im relevanten Wellenlängenbereich selbst fluoreszieren. Die Forderung nach inerten dentalen Abform-oder Filmmaterialien Trägermaterialien im Sinne der diagnostischen Zielsetzung ist für den Fachmann trivial und kann vom Fachmann problemlos beachtet werden.

Die Erfindung wird nachfolgend durch Beispiele näher erläutert, ohne dass sie durch diese beschränkt werden soll.

### Anwendungsbeispiel 1

### Nachweis von Arg-Gingipain über eine Polyetherabformmasse

In einem laborüblichen Dreifingerkneter wurde eine Basispaste hergestellt, indem bis zur Homogenität 53,2 Gewichtsteile eines Aziridinopolyethers, der gemäß Beispiel 12 der DE-PS-17 45 810 erhalten wurde, mit 18,1 g eines hydrierten Palmöls und 6,4 Gewichtsteilen Dibenzyltoluol vermischt wurden. Diese Masse wurde mit 11,8 Teilen eines Copolymers aus Ethylenoxid- und Tetramethylenoxideinheiten einer mittleren molaren Masse von 6500, sowie 0,1 Teilen Laurylimidazol und 5,0 Teilen eines Blockcopolymers aus Ethylenoxid- und Propylenoxideinheiten mit einer mittleren Molmasse von 3500 vereinigt. Diese Masse wurde anschließend mit 5,3 Gewichtsteilen Kieselgur vermischt.

Eine Katalysatorpaste wurde durch Homogenisieren von 33,8 Gewichtsteilen Acetyltributylcitrat mit 14,1 Teilen Ethylenoxid-Propylenoxid-Blockcopolymer und 19,0 Teilen eines Sulfoniumsalzes vermischt, das gemäß Beispiel 31 der DE-PS-25 15 593 erhalten wurde. Diese Masse wurde vereinigt mit 11 Teilen Kieselgur und 20,5 Teilen pyrogener Kieselsäure sowie 1 Teil Titandioxid. Anschließend wurden als Puffersubstanzen 0,7 g Tris(hydroxymethyl)aminomethan, 0,8 g Glycylglycin und als Substrat 200 µg N-t-Boc-Val-Pro-Arg-7-Amido-4-methylcumarin zugegeben.

Basis- und Katalysatorpaste wurden im Volumenverhältnis 5:1 vermischt und härteten nach ca. 8 Minuten zu einem homogenen Gummi aus. Eine Dotierung der Oberfläche dieses Gummis während der Abbindephase mit 2 µl Arg-Gingipain-haltiger Lösung (Stammlösung: 0,5 mg/ml Arg-Gingipain in 200 mM Tris(hydroxymethyl)aminomethan pH 7,6) ergab nach wenigen Minuten an dieser Stelle eine intensiv blaue Fluoreszenzemission bei einer Anregungswellenlänge von 360 nm.

### Anwendungsbeispiel 2

### Nachweis von Arg-Gingipain auf Alginatprüfkörpern

Zu 10 g Alginat (Palgat Plus Quick, Fa. ESPE Dental AG) wurden 20 ml Lösung, enthaltend 0,12 g Tris(hydroxymethyl)aminomethan, 100 µg N-t-Boc-Val-Pro-Arg-7-Amido-4-methylcumarin, pH 7,6, gegeben und mit einem breiten Kunstoffspatel innerhalb 1 min zu einer homogenen Masse geknetet. Während der Abbindephase wurde der Alginatprüfkörper mit 2 µl Arg-Gingipain-haltiger Lösung (Stammlösung: 0,5 mg/ml Arg-Gingipain in 200 mM Tris-(hydroxymethyl)aminomethan pH 7,6) dotiert. An dieser Stelle konnte nach 5 min eine intensiv blaue Fluoreszenzemission bei einer Anregungswellenlänge von 360 nm beobachtet werden.

### Anwendungsbeispiel 3

### Nachweis von Arg-Gingipain über eine Alginatabformmasse in Zahnfleischtaschen

Zu 20 g Alginat (Palgat Plus Quick, Fa. ESPE Dental AG) wurden 40 ml Lösung, enthaltend 0,24 g Tris(hydroxymethyl)aminomethan, 0,26 g Glycylglycin, 200 µg N-t-Boc-Val-Pro-Arg-7-Amido-4-methylcumarin gegeben und mit einem breitem Kunstoffspatel innerhalb 1 min zu einer homogenen Masse geknetet. Die Alginatmasse wurde in einen handelsüblichen Abformlöffel eingebracht und am Ober- oder Unterkiefer eines Parodontitis-Patienten für 5 min plaziert. Intensiv blaue Fluoreszenzemissionen konnte bei einer Anregungswellenlänge von 360 nm an einzelnen Zahnfleischtaschenrändern beobachtet werden.

### Anwendungsbeispiel 4

### Nachweis von Milchsäure auf Alginatprüfkörpern

Zu 5 g Alginat wurden 10 ml Lösung, enthaltend 0,065 g Glycylglycin, 0,06 g Tris-(hydroxymethyl)aminomethan, 9 mg NAD, 0,23 mg Phenazinmethosulfat, 0,75 mg 3-(4,5-Dimethylthiazolyl-2)-2,5-diphenyltetrazoliumbromid (MTT), 463 Units Lactatdehydrogenase aus Schweineherz, gegeben und mit einem breitem Spatel innerhalb 1 min zu einer homogenen Masse geknetet. Der Alginatprüfkörper wurde mit 5 µl einer 10 mM Calactat Lösung in 100 mM Tris-(hydroxymethy)aminomethan, pH 9,0, dotiert. Nach 4 min war an der Dotierungstelle die Entwicklung einer blauen Färbung zu beobachten.

### Anwendungsbeispiel 5

### Bestimmung der Milchsäurebildung über eine Alginatabformmasse auf Zähnen

Zu 20 g Alginat werden 40 ml Lösung, enthaltend 0,26 g Glycylglycin, 0,24 g Tris-(hydroxymethyl)aminomethan, 36 mg NAD, 0,9 mg Phenazinmethosulfat, 3 mg 3-(4,5-Dimethylthiazolyl-2)-2,5-diphenyltetrazoliumbromid (MTT), 1850 Units Lactatdehydrogenase aus Schweineherz, gegeben und mit einem breitem Spatel innerhalb 1 min zu einer homogenen Masse geknetet. Die Alginatmasse wird in einen handelsüblichen Abformlöffel eingebracht und am Ober- oder Unterkiefer eines Patienten plaziert. Der Patient sollte zuvor die Zähne geputzt und mit einer 1 %igen Saccharose-Lösung gespült haben. Nach 4 min wird der Abformlöffel entnommen. Stellen mit Milchsäurebildung sind an der entstehenden blauen Färbung zu erkennen.

## Patentansprüche

1. Verformbares, härtbares oder filmbildendes dentales Abform- oder Filmmaterial, **dadurch gekennzeichnet, dass** es für die orts- und stoffspezifische intraorale Diagnose diagnostisch nutzbare Zusatzstoffe enthält, die ohne Kultivierungsschritt zu einem diagnostischen Ergebnis führen, wobei es sich bei den diagnostisch wirksamen Zusatzstoffen nicht um Modifikationssubstanzen handelt, die die intraoral ablaufenden Prozesse modifizieren.

2. Dentales Abform- oder Filmmaterial nach Anspruch 1, **dadurch gekennzeichnet, dass** es diagnostisch nutzbare Zusatzstoffe zum intraoralen ortsspezifischen Nachweis von pathogenen Substanzen und/oder von Mikroorganismen oder zum intraoralen ortsspezifischen Nachweis von Substanzen, die auf Munderkrankungen oder Heilungsprozesse hinweisen, enthält.

3. Dentales Abform- oder Filmmaterial nach einem der Ansprüche 1 oder 2, **dadurch gekennzeichnet, dass** die diagnostisch nutzbaren Zusatzstoffe in mikroverkapselter Form vorliegen.

4. Dentales Abform- oder Filmmaterial nach einem der Ansprüche 1 bis 3, **dadurch gekennzeichnet, dass** mindestens soviel diagnostische Zusatzstoffe enthalten sind, dass ein diagnostisches Signal wahrgenommen werden kann.

5. Dentales Abform- oder Filmmaterial nach einem der Ansprüche 1 bis 4, **dadurch gekennzeichnet, dass** die diagnostischen Zusatzstoffe in einer Menge von 0,0001 bis 10 Gew.-%, bevorzugt 0,01 bis 1 Gew.-% enthalten sind.

6. Dentales Abform- oder Filmmaterial nach einem der Ansprüche 1 bis 5, **dadurch gekennzeichnet, dass** es aus einer der folgenden Gruppen ausgewählt ist:
(i) Abformmassen oder Filme auf Silikon-, Polyethersilikon-, Polyether-, Alginat- oder Hydrokolloidbasis,
(ii) Kunststoffe aus der Gruppe Polyethylene, Polypropylene, Poly(meth)acrylate, Polyurethane, Polycarbonate, Polysulfid, Polyvinylchloride oder Kautschuk,
(iii) Hydrogele auf Polyvinylpyrrolidon- oder Polyvinylalkoholbasis, oder
(iv) dentale Gipszubereitungen.

7. Dentales Abform- oder Filmmaterial nach Anspruch 6, **dadurch gekennzeichnet, dass** es eine Abformmasse auf N-Alkylaziridinopolyetherbasis ist.

8. Dentales Abform- oder Filmmaterial nach Anspruch 7, **dadurch gekennzeichnet, dass** es umfasst:
(A) 30 bis 96,9999 Gew.-% mindestens eines N-Alkylaziridinopolyethers mit
einer Molmasse im Bereich von 1.000 bis 20.000 g/Mol und einer Aziridinoäquivalentmasse im Bereich von 500 bis 8.000 g/Äquivalent,
(B) 1 bis 10 Gew.-% Startersubstanzen, die geeignet sind, die Aushärtung der N-Alkylaziridinopolyether zu bewirken,
(C) 1 bis 50 Gew.-% organische Verdünnungsmittel,
(D)1 bis 50 Gew.-% Modifikatoren, einschließlich Füllstoffen, Farbstoffe, Pigmente, Thixotropiemittel, Fließverbesserer, polymere Eindicker, oberflächenaktiven Substanzen, Geruchsstoffe und Geschmacksstoffe,
(E) 0,0001 bis 10 Gew.-% diagnostische Zusatzstoffe.

9. Verfahren zur Herstellung von Abbildungen für intraorale orts- und stoffspezifische Diagnosezwecke, **dadurch gekennzeichnet, dass** diagnostisch nutzbare Zusatzstoffe auf verformbare, härtbare oder filmbildende dentale Abform- oder Filmmaterialien, die keine diagnostisch nutzbaren Zusatzstoffe enthalten, in einer solchen Menge aufgebracht werden, dass ein diagnostisches Signal wahrgenommen werden kann, wobei die Zusatzstoffe ohne Kultivierungsschritt zu einem diagnostischen Ergebnis führten, und
wobei es sich bei den diagnostisch wirksamen Zusatzstoffen nicht um Modifikationssubstanzen handelt, die die intraoral ablaufenden Prozesse modifizieren.

10. Verfahren nach Anspruch 9, **dadurch gekennzeichnet, dass** diagnostisch nutzbare Zusatzstoffe auf verformbare, härtbare oder filmbildende dentale Abform- oder Filmmaterialien, die keine diagnostisch nutzbaren Zusatzstoffe enthalten, in einer Menge aufgebracht werden, dass ein diagnostisches Signal in Form des intraoralen orts- und stoffspezifischen Nachweises von pathogenen Substanzen und/oder von Mikroorganismen oder in Form des intraoralen orts- und stoffspezifischen Nachweises von Substanzen, die auf Munderkrankungen oder Heilungsprozesse hinweisen, wahrgenommen werden kann.

11. Verfahren nach einem der Ansprüche 9 oder 10, **dadurch gekennzeichnet, dass** die diagnostisch nutzbaren Zusatzstoffe in mikroverkapselter Form vorliegen.

12. Verfahren nach einem der Ansprüche 9 bis 11, **dadurch gekennzeichnet, dass** die diagnostischen Zusatzstoffe in einer Menge von 0,0001 bis 10 Gew.-%, bevorzugt 0,01 bis 1 Gew.-%, eingesetzt werden.

13. Verfahren nach einem der Ansprüche 9 bis 12, **dadurch gekennzeichnet, dass** das dentale Abform- oder Filmmaterial aus einer der folgenden Gruppen ausgewählt wird:
(i) Abformmassen oder Filme auf Silikon-, Polyethersilikon-, Polyether-, Alginat- oder Hydrokolloidbasis,
(ii) Kunststoffe aus der Gruppe Polyethylene, Polypropylene, Poly(meth)acrylate, Polyurethane, Polycarbonate, Polysulfid, Polyvinylchloride oder Kautschuk,
(iii) Hydrogele auf Polyvinylpyrrolidon- oder Polyvinylalkoholbasis, oder
(iv) dentale Gipszubereitungen.

14. Verfahren nach Anspruch 13, **dadurch gekennzeichnet, dass** als dentales Abform- oder Filmmaterial eine Abformmasse auf N-Alkylaziridinopolyetherbasis ausgewählt wird.

15. Verfahren nach Anspruch 14, **dadurch gekennzeichnet, dass** das dentale Abform- oder Filmmaterial umfasst:
(A)30 bis 96,9999 Gew.-% mindestens eines N-Alkylaziridinopolyethers mit einer Molmasse im Bereich von 1.000 bis 20.000 g/Mol und einer Aziridinoäquivalentmasse im Bereich von 500 bis 8.000 g/Äquivalent,
(B)1 bis 10 Gew.-% Startersubstanzen, die geeignet sind, die Aushärtung der N-Alkylaziridinopolyether zu bewirken,
(C)1 bis 50 Gew.-% organische Verdünnungsmittel,
(D)1 bis 50 Gew.-% Modifikatoren, einschließlich Füllstoffen, Farbstoffe, Pigmente, Thixotropiemittel, Fließverbesserer, polymere Eindicker, oberflächenaktiven Substanzen, Geruchsstoffe und Geschmacksstoffe,
(E) 0,0001 bis 10 Gew.-% diagnostische Zusatzstoffe.

16. Verwendung eines verformbaren, härtbaren oder filmbildenden dentalen Abform- oder Filmmaterials, das diagnostisch wirksame Zusatzstoffe enthält, zur gleichzeitigen multiplen sowie orts- und stoffspezifischen intraoralen Befunderhebung, wobei mit dem Abform- oder Filmmaterial eine Abdrucknahme erfolgt und gegebenenfalls weitere diagnostisch wirksame Zusatzstoffe aufgetragen werden,
oder
Verwendung eines verformbaren, härtbaren oder filmbildenden dentalen Abform- oder Filmmaterials, das keine diagnostisch wirksamen Zusatzstoffe enthält, zur gleichzeitigen multiplen sowie orts- und stoffspezifischen intraoralen Befunderhebung, wobei mit dem Abform- oder Filmmaterial eine Abdrucknahme erfolgt und diagnostisch wirksame Zusatzstoffe aufgetragen werden,
wobei die diagnostischen Zusatzstoffe ohne Kultivierungsschritt zu einem diagnostischen Ergebnis führen und es sich bei den diagnostisch wirksamen Zusatzstoffen nicht um Modifikationssubstanzen handelt, die die intraoral ablaufenden Prozesse modifizieren.

17. Verfahren bzw. Verwendung nach einem der Ansprüche 9 bis 16, **dadurch gekennzeichnet, dass** das er haltene Diagnoseergebnis vom Abdruck auf einen Positivabdruck, bevorzugt auf Gips, Hydrogele, Modellsilikone, übertragen wird.

18. Dentales Abform- oder Filmmaterial nach einem der Ansprüche 1 bis 8 oder Verfahren bzw. Verwendung nach einem der Ansprüche 9 bis 17, **dadurch gekennzeichnet, dass** die diagnostisch wirksamen Zusätze ausgewählt sind aus der Gruppe, bestehend aus: Farbstoffindikatoren, Redoxindikatoren, Fluoreszenzindikatoren, Chemolumineszenzindikatoren, Vitalitätsindikatoren, Antikörper und Enzyme.

## Claims

1. Mouldable, curable or film-forming dental impression or film material, **characterized in that** it contains diagnostically utilizable additives for site- and substance-specific intraoral diagnosis, which lead to a diagnostic result without a culturing step, where the diagnostically active additives are not modification substances that modify the processes proceeding intraorally.

2. Dental impression or film material according to Claim 1, **characterized in that** it contains diagnostically utilizable additives for the intraoral site-specific detection of pathogenic substances and/or of microorganisms or for the intraoral site-specific detection of substances that indicate oral diseases or healing processes.

3. Dental impression or film material according to one of Claims 1 or 2, **characterized in that** the diagnostically utilizable additives are present in microencapsulated form.

4. Dental impression or film material according to one of Claims 1 to 3, **characterized in that** at least so many diagnostic additives are contained that a diagnostic signal can be observed.

5. Dental impression or film material according to one of Claims 1 to 4, **characterized in that** the diagnostic additives are contained in an amount from 0.0001 to 10% by weight, preferably 0.01 to 1% by weight.

6. Dental impression or film material according to one of Claims 1 to 5, **characterized in that** it is selected from one of the following groups:
(i) impression compositions or films based on silicone, polyether silicone, polyether, alginate or hydrocolloid,
(ii) plastics from the group consisting of polyethylenes, polypropylenes, poly- (meth)acrylates, polyurethanes, poly-carbonates, polysulphide, polyvinyl chlorides or rubber,
(iii) hydrogels based on polyvinylpyrrolidone or polyvinyl alcohol, or
(iv) dental gypsum preparations.

7. Dental impression or film material according to Claim 6, **characterized in that** it is an impression composition based on N-alkylaziridinopolyethers.

8. Dental impression or film material according to Claim 7, **characterized in that** it comprises:
(A) 30 to 96.9999% by weight of at least one N-alkylaziridinopolyether having a molar mass in the range from 1000 to 20,000 g/mol and an aziridino equivalent mass in the range from 500 to 8000 g/equivalent,
(B) 1 to 10% by weight of starter substances, which are suitable for bringing about the curing of the N-alkylaziridinopolyether,
(C) 1 to 50% by weight of organic diluents,
(D) 1 to 50% by weight of modifiers, including fillers, dyes, pigments, thixotropic agents, flow improvers, polymeric thickeners, surface-active substances, odorants and flavourings,
(E) 0.0001 to 10% by weight of diagnostic additives.

9. Method for the production of images for intraoral site- and substance-specific diagnosis purposes, **characterized in that** diagnostically utilizable additives are applied to mouldable, curable or film-forming dental impression or film materials that contain no diagnostically utilizable additives, in an amount such that a diagnostic signal can be observed, the additives leading to a diagnostic result without a culturing step, and where the diagnostically active additives are not modification substances that modify the processes proceeding intraorally.

10. Method according to Claim 9, **characterized in that** diagnostically utilizable additives are applied to mouldable, curable or film-forming dental impression or film materials that contain no diagnostically utilizable additives in an amount that a diagnostic signal in the form of the intraoral site- and substance-specific detection of pathogenic substances and/or of microorganisms or in the form of the intraoral site- and substance-specific detection of substances that indicate oral diseases or healing processes can be observed.

11. Method according to one of Claims 9 or 10, **characterized in that** the diagnostically utilizable additives are present in micro-encapsulated form.

12. Process according to one of Claims 9 to 11, **characterized in that** the diagnostic additives are employed in an amount from 0.0001 to 10% by weight, preferably 0.01 to 1% by weight.

13. Method according to one of Claims 9 to 12, **characterized in that** the dental impression or film material is selected from one of the following groups:
(i) impression compositions or films based on silicone, polyether silicone, polyether, alginate or hydrocolloid,
(ii) plastics from the group consisting of polyethylenes, polypropylenes, poly- (meth)acrylates, polyurethanes, poly-carbonates, polysulphide, polyvinyl chlorides or rubber,
(iii) hydrogels based on polyvinylpyrrolidone or polyvinyl alcohol, or
(iv) dental gypsum preparations.

14. Method according to Claim 13, **characterized in that** an impression composition based on N-alkyl-aziridinopolyether is selected as the dental impression or film material.

15. Method according to Claim 14, **characterized in that** the dental impression or film material comprises:
(A) 30 to 96.9999% by weight of at least one N-alkylaziridinopolyether having a molar mass in the range from 1000 to 20,000 g/mol and an aziridino equivalent mass in the range from 500 to 8000 g/equivalent,
(B) 1 to 10% by weight of starter substances, which are suitable for bringing about the curing of the N-alkylaziridinopolyether,
(C) 1 to 50 % by weight of organic diluents,
(D) 1 to 50% by weight of modifiers, including fillers, dyes, pigments, thixotropic agents, flow improvers, polymeric thickeners, surface-active substances, odorants and flavourings,
(E) 0.0001 to 10% by weight of diagnostic additives.

16. Use of a mouldable, curable or film-forming dental impression or film material that contains diagnostically active additives, for simultaneous multiple and site- and substance-specific intraoral diagnosis, impression taking being carried out using the impression or film material and further diagnostically active additives optionally being applied,
or
use of a mouldable, curable or film-forming dental impression or film material that contains no diagnostically active additives for simultaneous multiple and site- and substance-specific oral diagnosis, impression taking being carried out with the impression or film material and diagnostically active additives being applied,
the diagnostic additives leading to a diagnostic result without a culturing step and the diagnostically active additives not being modification substances that modify the processes proceeding intraorally.

17. Method or use according to one of Claims 9 to 16, **characterized in that** the diagnosis result obtained from the impression is transferred to a positive impression, preferably on gypsum, hydrogels or modelling silicones.

18. Dental impression or film material according to one of Claims 1 to 8 or method or use according to one of Claims 9 to 17, **characterized in that** the diagnostically active additives are selected from the group consisting of: dye indicators, redox indicators, fluorescent indicators, chemo-luminescent indicators, vitality indicators, antibodies and enzymes.

## Revendications

1. Matériau de film ou de moulage dentaire, déformable, durcissable ou filmogène, **caractérisé en ce qu'**il contient des additifs utilisables pour le diagnostic pour le diagnostic intra-oral spécifique de localisation et de matière, qui conduisent à un résultat de diagnostic sans étape de culture, les additifs efficaces pour le diagnostic ne consistant pas en des substances modificatrices qui modifient les processus se déroulant à l'intérieur de la cavité buccale.

2. Matériau de film ou de moulage dentaire selon la revendication 1, **caractérisé en ce qu'**il contient des additifs utilisables pour le diagnostic pour la détection intra-orale, spécifique de localisation, de substances pathogènes et/ou de micro-organismes ou pour la détection intra-orale, spécifique de localisation, de substances qui indiquent des maladies de la bouche ou des processus de guérison.

3. Matériau de film ou de moulage dentaire selon la revendication 1 ou 2, **caractérisé en ce que** les additifs utilisables pour le diagnostic se trouvent sous forme micro-encapsulée.

4. Matériau de film ou de moulage dentaire selon l'une quelconque des revendications 1 à 3, **caractérisé en ce qu'**au moins suffisamment d'additifs de diagnostic sont contenus pour qu'un signal de diagnostic puisse être décelé.

5. Matériau de film ou de moulage dentaire selon l'une quelconque des revendications 1 à 4, **caractérisé en ce que** les additifs de diagnostic sont contenus en une quantité de 0,0001 à 10 % en poids, de préférence de 0,01 à 1 % en poids.

6. Matériau de film ou de moulage dentaire selon l'une quelconque des revendications 1 à 5, **caractérisé en ce qu'**il est choisi dans l'un des groupes suivants :
(i) matières de moulage ou films à base de silicone, polyéther-silicone, polyéther, alginate ou hydrocolloïde,
(ii) matières plastiques choisies dans le groupe des polyéthylènes, polypropylènes, poly(méth)acrylates, polyuréthannes, polycarbonates, polysulfure, poly(chlorure de vinyle)s ou caoutchouc,
(iii) hydrogels à base de polyvinylpyrrolidone ou poly(alcool vinylique), ou
(iv) préparations dentaires à base de plâtre.

7. Matériau de film ou de moulage dentaire selon la revendication 6, **caractérisé en ce qu'**il s'agit d'une matière de moulage à base de N-alkylaziridinopolyéther.

8. Matériau de film ou de moulage dentaire selon la revendication 7, **caractérisé en ce qu'**il comprend :
(A) 30 à 96,9999 % en poids d'au moins un N-alkylaziridinopolyéther ayant une masse molaire dans la plage de 1 000 à 20 000 g/mole et une masse d'équivalent aziridino dans la plage de 500 à 8 000 g/équivalent,
(B) 1 à 10 % en poids de substances d'amorçage, qui sont appropriées pour provoquer le durcissement des N-alkylaziridinopolyéthers,
(C) 1 à 50 % en poids de diluants organiques,
(D) 1 à 50 % en poids de modificateurs, incluant charges, colorants, pigments, agents de thixotropie, agents améliorant l'écoulement, épaississants polymères, substances tensioactives, parfums et arômes,
(E) 0,0001 à 10 % en poids d'additifs de diagnostic.

9. Procédé pour la production d'images à des fins de diagnostic spécifique de localisation et de matière, **caractérisé en ce qu'**on applique des additifs utilisables pour le diagnostic sur des matériaux de film ou de moulage dentaires, déformables, durcissables ou filmogènes, qui ne contiennent pas d'additifs utilisables pour le diagnostic, en une quantité telle qu'un signal de diagnostic puisse être décelé, les additifs conduisant à un résultat de diagnostic sans étape de culture, et les additifs efficaces pour le diagnostic ne consistant pas en des substances modificatrices qui modifient les processus se déroulant à l'intérieur de la cavité buccale.

10. Procédé selon la revendication 9, **caractérisé en ce qu'**on applique des additifs utilisables pour le diagnostic sur des matériaux de film ou de moulage dentaires, déformables, durcissables ou filmogènes, qui ne contiennent pas d'additifs utilisables pour le diagnostic, en une quantité telle que puisse être décelé un signal de diagnostic sous forme de la détection intra-orale, spécifique de localisation et de matière, de substances pathogènes et/ou de micro-organismes ou sous forme de la détection intra-orale, spécifique de localisation et de matière, de substances qui indiquent des maladies de la bouche ou des processus de guérison.

11. Procédé selon l'une quelconque des revendications 9 ou 10, **caractérisé en ce que** les additifs utilisables pour le diagnostic se trouvent sous forme micro-encapsulée.

12. Procédé selon l'une quelconque des revendications 9 à 11, **caractérisé en ce que** les additifs de diagnostic sont utilisés en une quantité de 0,0001 à 10 % en poids, de préférence de 0,01 à 1 % en poids.

13. Procédé selon l'une quelconque des revendications 9 à 12, **caractérisé en ce que** le matériau de moulage ou de film dentaire est choisi dans l'un des groupes suivants :
(i) matières de moulage ou films à base de silicone, polyéther-silicone, polyéther, alginate ou hydrocolloïde,
(ii) matières plastiques choisies dans le groupe des polyéthylènes, polypropylènes, poly(méth)acrylates, polyuréthannes, polycarbonates, polysulfure, poly(chlorure de vinyle)s ou caoutchouc,
(iii) hydrogels à base de polyvinylpyrrolidone ou poly(alcool vinylique), ou
(iv) préparations dentaires à base de plâtre.

14. Procédé selon la revendication 13, **caractérisé en ce que** comme matériau de moulage ou de film dentaire on choisit une matière de moulage à base de N-alkylaziridinopolyéther.

15. Procédé selon la revendication 14, **caractérisé en ce que** le matériau de moulage ou de film dentaire comprend :
(A) 30 à 96,9999 % en poids d'au moins un N-alkylaziridinopolyéther ayant une masse molaire dans la plage de 1 000 à 20 000 g/mole et une masse d'équivalent aziridino dans la plage de 500 à 8 000 g/équivalent,
(B) 1 à 10 % en poids de substances d'amorçage, qui sont appropriées pour provoquer le durcissement des N-alkylaziridinopolyéthers,
(C) 1 à 50 % en poids de diluants organiques,
(D) 1 à 50 % en poids de modificateurs, incluant charges, colorants, pigments, agents de thixotropie, agents améliorant l'écoulement, épaississants polymères, substances tensioactives, parfums et arômes,
(E) 0,0001 à 10 % en poids d'additifs de diagnostic.

16. Utilisation d'un matériau de film ou de moulage dentaire, déformable, durcissable ou filmogène, qui contient des additifs efficaces pour le diagnostic, pour l'examen intra-oral multiple simultané ainsi que spécifique de localisation et de matière, une prise d'empreinte s'effectuant avec le matériau de moulage ou de film et éventuellement d'autres additifs efficaces pour le diagnostic étant appliqués,
ou
utilisation d'un matériau de film ou de moulage dentaire, déformable, durcissable ou filmogène, qui ne contient pas d'additifs efficaces pour le diagnostic, pour l'examen intra-oral multiple simultané ainsi que spécifique de localisation et de matière, une prise d'empreinte s'effectuant avec le matériau de moulage ou de film et des additifs efficaces pour le diagnostic étant appliqués,
les additifs de diagnostic conduisant à un résultat de diagnostic sans étape de culture et les additifs efficaces pour le diagnostic ne consistant pas en des substances modificatrices qui modifient les processus se déroulant à l'intérieur de la cavité buccale.

17. Procédé ou utilisation selon l'une quelconque des revendications 9 à 16, **caractérisé**(e) en ce que le résultat du diagnostic obtenu est transféré de l'empreinte sur une empreinte positive, de préférence sur du plâtre, des hydrogels, des silicones de modelage.

18. Matériau de film ou de moulage dentaire selon l'une quelconque des revendications 1 à 8 ou procédé ou utilisation selon l'une quelconque des revendications 9 à 17, **caractérisé**(e) en ce que les additifs efficaces pour le diagnostic sont choisis parmi : des indicateurs à base de colorants, des indicateurs redox, des indicateurs fluorescents, des indicateurs chimiluminescents, des indicateurs de vitalité, des anticorps et des enzymes.
